(19) Europäisches Patentamt
European Patent Office
Office européen des brevets

(11) EP 4 067 475 A1

(12) **EUROPEAN PATENT APPLICATION**
published in accordance with Art. 153(4) EPC

(43) Date of publication:
**05.10.2022 Bulletin 2022/40**

(21) Application number: **20891690.8**

(22) Date of filing: **24.11.2020**

(51) International Patent Classification (IPC):
***C12N 1/20** (2006.01)* ***C12N 1/16** (2006.01)*

(52) Cooperative Patent Classification (CPC):
**C12N 1/16; C12N 1/20**

(86) International application number:
**PCT/JP2020/043557**

(87) International publication number:
**WO 2021/106822 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.11.2019 JP 2019212464**
**25.11.2019 JP 2019212465**

(71) Applicant: **Kao Corporation**
**Chuo-ku**
**Tokyo 103-8210 (JP)**

(72) Inventors:
- **KIJITORI Hiroki**
**Wakayama-shi, Wakayama 640-8580 (JP)**
- **IZAWA Yoshifumi**
**Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

(54) **METHOD FOR CONTROLLING BRANCHED FATTY ACID METABOLISM**

(57) The present invention is a method for controlling the metabolism of a branched fatty acid including, bringing (a) one or more compounds selected from a compound represented by the following general formula (1) into contact with a microorganism to control the metabolism of a branched fatty acid of the microorganism,

$$RO\text{-}(C_2H_4O)_n\text{-}H \qquad (1)$$

wherein R is a linear aliphatic hydrocarbon group with 9 or more and 16 or less carbons, and n is 0 or 1.

EP 4 067 475 A1

## Description

Field of the Invention

The present invention relates to a method for controlling the metabolism of a branched fatty acid, and an agent for controlling the metabolism of a branched fatty acid.

The present invention relates to a method for controlling the metabolism of a branched fatty acid in the fields of, for example, biology, biochemistry, physiological science and products for consumer use.

Background of the Invention

In the field of products for consumer use, for example, consumers have recently been more concerned about their living environment and desirous to reduce or eliminate unpleasant odors around them. Underwear, towels, handkerchiefs and other textile products which come into direct contact with human skin, or textile products which may absorb sweat, keratin and the like containing sebum or to which they may adhere may have a peculiar odor.

Conventionally, methods of suppressing the generation of odors by controlling metabolic reactions to substances causative of unpleasant odors occurring within the cells of bacteria (microorganisms) causing the production of substances causative of unpleasant odors have been studied.

For example, as an agent for suppressing the conversion of a sebum stain component to 4-methyl-3-hexenoic acid, which is one of the substances causing a sour laundry odor, JP-A 2012-127012 discloses a ketone compound having a specific structure.

In addition, as an agent for inhibiting activity of β -glucuronidase to suppress the generation of a urine odor, WO-A 2009/037861 discloses a macrocyclic ketone compound having a specific structure.

Further, there are also known techniques that use an organic carboxylic acid having a specific structure such as benzoic acid or the like to kill microorganisms, thereby suppressing the generation of odors.

Further, as one of the microorganism controlling methods, JP-A 2009-078987 discloses, in connection with the suppression of microbial biofilm formation, a method for suppressing biofilm formation comprising bringing a microorganism into contact with a specific alcohol having a linear or branched alkyl group or alkenyl group with 8 to 14 carbons or an EO adduct thereof in a specific concentration.

Summary of the Invention

For example, the following problem lies in products for consumer use. It is known that various microorganisms produce substances causative of unpleasant odors by metabolizing branched fatty acids as substrates. If such microorganisms are killed, unpleasant odors can be suppressed, but useful bacteria present in the environment, the body or the like might also be killed. In other words, from the viewpoint of coexistence with the environment or maintaining healthy skin, suppressing the generation of unpleasant odors especially without killing microorganisms may need to be studied.

In view of the foregoing, a problem to be solved by the present invention is to provide a method for controlling the metabolism of a branched fatty acid within a microorganism, and a method for controlling the metabolism of a branched fatty acid to control the production amount of a product of the metabolism of the branched fatty acid.

The present invention provides, for example, in the field of products for consumer use, a method for controlling the metabolism of a branched fatty acid capable of controlling the production of substances causative of unpleasant odors from the metabolism of branched fatty acids as substrates by microorganisms causing unpleasant odors without killing the microorganisms.

The present invention relates to a method for controlling the metabolism of a branched fatty acid including, bringing (a) one or more compounds selected from a compound represented by the following general formula (1) into contact with a microorganism to control the metabolism of a branched fatty acid of the microorganism,

$$RO\text{-}(C_2H_4O)_n\text{-}H \qquad (1)$$

wherein R is a linear aliphatic hydrocarbon group with 9 or more and 16 or less carbons, and n is 0 or 1.

In addition, the present invention relates to a method for controlling the metabolism of a branched fatty acid including, bringing (a) one or more compounds selected from a compound represented by the following general formula (1) [hereinafter referred to as component (a)] and (b) an inhibiting agent for fatty acid synthases [hereinafter referred to as component (b)] into contact with a microorganism to control the metabolism of a branched fatty acid of the microorganism,

$$RO\text{-}(C_2H_4O)_n\text{-}H \qquad (1)$$

wherein R is a linear aliphatic hydrocarbon group with 9 or more and 16 or less carbons, and n is 0 or 1.

In addition, the present invention relates to an agent for controlling the metabolism of a branched fatty acid in a microorganism containing, (a) one or more compounds selected from the compound represented by the general formula (1).

In addition, the present invention relates to an agent for controlling the metabolism of a branched fatty acid in a microorganism containing, (a) one or more compounds selected from the compound represented by the general formula (1) and (b) an inhibiting agent for fatty acid synthases.

The method or agent for controlling the metabolism of a branched fatty acid of the present invention is capable of controlling the degree of metabolism of branched fatty acids within microorganisms. In particular, the degree of β oxidation of branched fatty acids within microorganisms or the degree of incorporation of branched fatty acids into microorganisms can be controlled.

Embodiments of the Invention

The present inventors consider a mechanism for controlling the metabolism of branched fatty acids by the method or agent for controlling the metabolism of a branched fatty acid to be as follows, but it is not necessarily limited thereto.

Branched fatty acids are considered to be metabolized through the following processes: a process in which they pass through the cell membranes of microorganisms; and a process in which the branched fatty acids incorporated inside the cell membranes are metabolized by β-oxidation to produce metabolites. The compound represented by the general formula (1) of component (a) is considered to be incorporated into microorganisms faster than corresponding fatty acids.

The compound represented by the general formula (1) incorporated undergoes oxidation by alcohol dehydrogenase and subsequently modification by acyl-CoA synthetase within bacterial bodies to be converted into a fatty acid-CoA corresponding to the general formula (1). The produced fatty acid-CoA is considered to act on FadR, the transcription regulator protein for fatty acid metabolic/synthetic enzymes, to suppress the incorporation of branched fatty acids present outside bacterial bodies into the microorganisms and the metabolism thereof. The fact that the metabolism of branched fatty acids is controlled by incorporating the compound represented by the general formula (1) into microorganisms and controlling the metabolism has not been conventionally known in the art. A common method for controlling metabolism by microorganisms may be, for example, a method of decreasing the number of microorganisms to reduce the metabolism of branched fatty acids. However, a method capable of adjusting the degree of metabolism of branched fatty acids within microorganisms while substantially maintaining the number of microorganisms like the present invention has not been known. Moreover, if FadR is activated, fatty acid elongases are activated to produce long-chain fatty acids and increase the amount of long-chain fatty acids within microorganisms. As the produced long-chain fatty acids are an inactivating factor for FadR, FadR is inactivated, resulting in the initiation of the incorporation/metabolism of extracellular fatty acids. When the inhibitor for fatty acid synthases of component (b) is used in the present invention, as component (b) inhibits reactions of fatty acid synthases and prevents FadR from being inactivated, the effect of component (a) of the present invention is enhanced. It is inferred that compounds not having a linear hydrocarbon group such as branched aliphatic alcohols are less likely to cause the processes from oxidation within bacterial bodies to the production of a fatty acid-CoA than the compound represented by the general formula (1) having a linear hydrocarbon group such as linear aliphatic alcohols, and cannot sufficiently control the incorporation of branched fatty acids into microorganisms or the metabolism thereof.

<Component (a)>

Component (a) used in the method of the present invention is one or more compounds selected from a compound represented by the following general formula (1) :

$$RO\text{-}(C_2H_4O)_n\text{-}H \qquad (1)$$

wherein R is a linear aliphatic hydrocarbon group with 9 or more and 16 or less carbons, and n is 0 or 1.

Commercially available products may be used as component (a). Component (a) can also be synthesized by a usual method for producing an alcohol or an ether. Two or more types of component (a) may be used.

In the general formula (1), R is a linear aliphatic hydrocarbon group with 9 or more and 16 or less carbons, and the carbon number is preferably 10 or more, more preferably 11 or more and further preferably 12 or more from the viewpoint of further controlling the metabolism of a branched fatty acid and particularly from the viewpoint of suppressing the metabolism of a branched fatty acid, and preferably 15 or less and more preferably 14 or less from the same

viewpoints. The linear aliphatic hydrocarbon group is preferably a linear alkyl group and preferably a linear primary alkyl group.

In the general formula (1), n is preferably 0 from the viewpoint of further controlling the metabolism of a branched fatty acid and particularly from the viewpoint of suppressing the metabolism of a branched fatty acid.

R in the general formula (1) is preferably one or more groups selected from a linear saturated aliphatic hydrocarbon group with 12 or 13 carbons and preferably one or more groups selected from a linear primary alkyl group with 12 or 13 carbons from the viewpoint of being excellent in the effect of controlling the metabolism of a branched fatty acid, particularly suppressing the metabolism of a branched fatty acid, and from the viewpoint of controlling the production amount of a product of the metabolism of a branched fatty acid, particularly suppressing the production amount of a product of the metabolism of a branched fatty acid, even in gram-positive bacteria and gram-negative bacteria, which are described later as examples of the microorganism.

The proportion of a compound in which R has 12 or 13 carbons in component (a) is preferably 30 mass% or more, more preferably 50 mass% or more, further preferably 60 mass% or more, furthermore preferably 70 mass% or more, furthermore preferably 80 mass% or more, furthermore preferably 90 mass% or more, furthermore preferably 95 mass% or more and furthermore preferably 97 mass% or more, and 100 mass% or less, or may be 100 mass% from the viewpoint of the effect per mass of component (a) of controlling the metabolism of a branched fatty acid, particularly suppressing the metabolism of a branched fatty acid.

The content of a compound in which R is a linear saturated aliphatic hydrocarbon group with 9 or more and 16 or less carbons in component (a) is preferably 50 mass% or more, further preferably 60 mass% or more, furthermore preferably 70 mass% or more, furthermore preferably 80 mass% or more, furthermore preferably 90 mass% or more, furthermore preferably 95 mass% or more and furthermore preferably 97 mass% or more, and 100 mass% or less, or may be 100 mass% from the viewpoint of the effect per mass of component (a) of controlling the metabolism of a branched fatty acid, particularly suppressing the metabolism of a branched fatty acid.

R is preferably one or more groups selected from a linear saturated aliphatic hydrocarbon group with 12 or 13 carbons from the viewpoint of controlling the metabolism of a branched fatty acid and particularly from the viewpoint of suppressing the metabolism of a branched fatty acid.

The content proportion of a compound in which R is a linear saturated aliphatic hydrocarbon group with 12 or 13 carbons in component (a) is preferably 50 mass% or more, further preferably 60 mass% or more, furthermore preferably 70 mass% or more, furthermore preferably 80 mass% or more, furthermore preferably 90 mass% or more, furthermore preferably 95 mass% or more and furthermore preferably 97 mass% or more, and 100 mass% or less, or may be 100 mass% from the viewpoint of the effect per mass of component (a) of controlling the metabolism of a branched fatty acid, particularly suppressing the metabolism of a branched fatty acid.

Specific examples of a compound of component (a) in which n in the general formula (1) is 0 include one or more compounds selected from 1-nonyl alcohol, 1-decanol, 1-undecanol, 1-dodecanol, 1-tridecanol, 1-tetradecanol, 1-pentadecanol and 1-hexadecanol. Component (a) is preferably one or more compounds selected from 1-decanol, 1-undecanol, 1-dodecanol, 1-tridecanol, 1-tetradecanol and 1-pentadecanol, more preferably one or more compounds selected from 1-dodecanol, 1-tridecanol and 1-tetradecanol, and further preferably one or more compounds selected from 1-dodecanol and 1-tridecanol from the viewpoint of controlling the metabolism of a branched fatty acid and particularly from the viewpoint of suppressing the metabolism of a branched fatty acid. In addition, component (a) is preferably one or more compounds selected from 1-dodecanol and 1-tridecanol from the viewpoint of controlling the metabolism of a branched fatty acid, particularly suppressing the metabolism of a branched fatty acid with a lower amount. Component (a) is preferably 1-dodecanol when it is used in combination with component (b) described later.

Specific examples of component (a) in which n in the general formula (1) is 1 include one or more compounds selected from ethylene glycol mono-1-dodecyl ether, ethylene glycol mono-1-tridecyl ether and ethylene glycol mono-1-tetradecyl ether. Component (a) in which n in the general formula (1) is 1 is preferably one or more compounds selected from ethylene glycol mono-1-dodecyl ether and ethylene glycol mono-1-tridecyl ether, and more preferably ethylene glycol mono-1-dodecyl ether from the viewpoint of controlling the metabolism of a branched fatty acid and particularly from the viewpoint of suppressing the metabolism of a branched fatty acid.

<Component (b)>

In the present invention, components (a) and (b) can be used. Component (b) is an inhibiting agent for fatty acid synthases. Fatty acids within the cells of microorganisms may be biosynthesized by fatty acid synthases. The following fatty acid synthases are generally known: Type 1 (FAS-I) present in cytoplasm; and Type II (FAS-II) present in mitochondria. Various compounds are found to inhibit fatty acid synthases, and they are suitable for use in the present invention. Selecting inhibiting agents for fatty acid synthases falls within the common technical knowledge of a person skilled in the art. A compound inhibiting a fatty acid synthase can be identified by testing the ability of the compound to inhibit activity of the fatty acid synthase by using a refined fatty acid synthase. For example, it can be identified by the

method described in Dils et al. (1975), Meth. Enzymol. volume 35, pages 74 to 83. Examples of inhibiting agents for fatty acid synthases are disclosed, for example, in WO-A 94/02108.

Examples of compounds preferable as component (b) include one or more compounds selected from the following components (b1), (b2), (b3) and (B4):

component (b1): a compound having a diphenyl ether group;
component (b2): thiolactomycin or a thiolactomycin derivative;
component (b3): cerulenin or a cerulenin derivative; and
component (b4): α-methylene-γ-butyrolactone or an α-methylene-γ-butyrolactone derivative.
Component (b1) is preferable from the viewpoint of being likelier to obtain the effect of suppressing the metabolism of a branched fatty acid with a lower amount.

[Component (b1)]

Component (b1) is a compound having a diphenyl ether group. Component (b1) is preferably a compound in which one or more hydrogen atoms of a phenyl group are substituted for by a hydroxyl group, and more preferably a compound in which two or more hydrogen atoms of a phenyl group are substituted for by a hydroxyl group and a chlorine group from the viewpoint of further enhancing the effect of inhibiting fatty acid synthases. More specifically, component (b1) is preferably one or more compounds selected from 4-4'-dichloro-2-hydroxydiphenyl ether (diclosan) and 2,4,4'-trichloro-2'-hydroxydiphenyl ether (triclosan).

[Component (b2)]

Component (b2) is thiolactomycin or a thiolactomycin derivative. A thiolactomycin derivative means a compound structurally related to thiolactomycin and possessing at least a measurable amount of fatty acid synthase inhibition activity. Non-limiting examples of thiolactomycin or a thiolactomycin derivative are described in Wang et al. (1984) Tetrahedron Lett., volume 25, pages 5243 to 5246, Oishi et al. (1982) J. Antibiotics, volume 35, pages 391 to 395, and Kremer et al. (2000) J. Bio. Chem., volume 275, pages 16857 to 16864.

[Component (b3)]

Component (b3) is cerulenin or a cerulenin derivative. It is structurally characterized as [2R,3S]-2,3-epoxy-4-oxo-7,10-trans,trans-dodecanoic acid amide.

A cerulenin derivative means a compound structurally related to cerulenin and possessing at least a measurable amount of fatty acid synthase inhibition activity. Examples of cerulenin and a cerulenin derivative include those described in Morisaki et al. (1992) Chem. Pharm. Bull., volume 40, pages 2945 to 2953, Shimazawa et al. (1992) Chem. Pharm. Bull., volume 40, pages 2954 to 2957 and US-B 5539132.

Alcohol

[Component (b4)]

Component (b4) is α-methylene-γ-butyrolactone or an α -methylene-γ-butyrolactone derivative. An α-methylene-γ-butyrolactone derivative means a compound structurally related to each α-methylene-γ-butyrolactone and having at least a measurable amount of fatty acid synthase inhibition activity. Examples of an α-methylene-γ-butyrolactone derivative include, for example, those described in US-B 5981575.

Component (b) is preferably one or more compounds selected from the above components (b1) and (b3), and more preferably one or more compounds selected from the above component (b1). More specifically, component (b) is preferably one or more compounds selected from 4-4'-dichloro-2-hydroxydiphenyl ether (diclosan), 2,4,4'-trichloro-2'-hydroxydiphenyl ether (triclosan) and cerulenin.

<Microorganism>

Microorganisms to which the present invention is directed are microorganisms having the ability to metabolize branched fatty acids, and they can be found in general microorganisms present in the environment. Examples thereof include, for example, one or more microorganisms selected from anaerobic bacteria, aerobic bacteria and yeast.

The present invention can be used for general microorganisms present in the environment. Examples of the microorganisms include, for example, one or more microorganisms selected from gram-negative bacteria, gram-positive

bacteria and yeast.

Examples of the gram-negative bacteria include bacteria of the genus Moraxella, bacteria of the genus Acinetobacter, bacteria of the genus Pseudomonas, bacteria of the genus Sphingomonas, bacteria of the genus Ralstonia, bacteria of the genus Cupriavidus, bacteria of the genus Psychrobacter, bacteria of the genus Serratia, bacteria of the genus Escherichia and bacteria of the genus Burkholderia.

Examples of bacteria of the genus Moraxella include, for example, Moraxella sp. and Moraxella osloensis. Examples of bacteria of the genus Acinetobacter include Acinetobacter radioresistens, Acinetobacter junii and Acinetobacter calcoaceticus. Examples of bacteria of the genus Pseudomonas include Pseudomonas alcaligenes. Examples of bacteria of the genus Sphingomonas include Sphingomonas yanoikuyae. Examples of bacteria of the genus Ralstonia include Ralstonia sp. Examples of bacteria of the genus Cupriavidus include Cupriavidus oxalaticus.

Examples of bacteria of the genus Psychrobacter include Psychrobacter pacificensis and Psychrobacter glacincola. Examples of bacteria of the genus Serratia include Serratia marcescens. Examples of bacteria of the genus Escherichia include Escherichia coli. Examples of bacteria of the genus Burkholderia include Burkholderia cepacia.

Examples of the gram-positive bacteria include bacteria of the genus Bacillus, bacteria of the genus Staphylococcus, bacteria of the genus Micrococcus and bacteria of the genus Corynebacterium.

Examples of bacteria of the genus Bacillus include Bacillus cereus and Bacillus subtilis. Examples of bacteria of the genus Staphylococcus include Staphylococcus aureus. Examples of bacteria of the genus Micrococcus include Micrococcus luteus, Micrococcus lylae and Micrococcus aloeverae. Examples of bacteria of the genus Corynebacterium include Corynebacterium xerosis.

Examples of the yeast include yeast of the genus Saccaromyces, yeast of the genus Rhodotorula and the like.

Examples of yeast of the genus Saccaromyces include Saccaromyces cerevisiae. Examples of yeast of the genus Rhodotorula include Rhodotorula mucilaginosa and

Rhodotorula slooffiae.

<Branched fatty acid>

The method for controlling the metabolism of a branched fatty acid of the present invention can be applied to metabolism in fatty acid synthesis systems in metabolic systems of microorganisms. For example, the method for controlling the metabolism of a branched fatty acid of the present invention may be a method for suppressing β oxidation of a branched fatty acid by a microorganism. β oxidation occurring in vivo is a metabolic pathway in fatty acid metabolism through which a fatty acid is oxidized to produce fatty acyl-CoA to retrieve acetyl-CoA, and qualifies as the first stage of the three stages of fatty acid metabolism (β-oxidation, citric acid circuit and electron transfer system).

Examples of the branched fatty acid include, for example, a branched fatty acid with 9 or more and 21 or less carbons. The branched fatty acid has preferably 11 or more, more preferably 13 or more, further preferably 15 or more and furthermore preferably 17 or more carbons from the viewpoint of further obtaining the effects of the present invention, and more preferably 19 or less carbons from the same viewpoint. In addition, the branched fatty acid is preferably a saturated or unsaturated branched fatty acid with 11 or more and 21 or less carbons, more preferably a saturated or unsaturated branched fatty acid with 13 or more and 19 or less carbons and furthermore preferably a saturated or unsaturated branched fatty acid with 15 or more and 19 or less carbons from the viewpoint of further obtaining the effects of the present invention.

Examples of the branched fatty acid include, for example, one or more branched fatty acids selected from a 10-methyl fatty acid, an iso fatty acid and an anteiso fatty acid.

In addition, examples of the branched fatty acid include a branched fatty acid having one or more methyl groups as branched chains.

Examples of the 10-methyl fatty acid include a 10-methyl fatty acid with 15 or more and 18 or less total carbons. Specific examples thereof include 10-methylpentadecanoic acid, 10-methylhexadecanoic acid, 1-methylheptadecanoic acid and 10-methyloctadecanoic acid.

An iso fatty acid is known as a fatty acid in which a methyl branch is attached to one carbon before the ω end of the fatty acid, or the carbon at ω-1. Examples of the iso fatty acid include an iso fatty acid with 10 or more and 24 or less total carbons. From the viewpoint of being likelier to be controlled by the metabolism controlling method of the present invention, examples thereof include an iso fatty acid with 12 or more and 18 or less total carbons.

An anteiso fatty acid is known as an anteiso-type branched fatty acid in which a methyl group substitutes for the third carbon counting from the terminal methyl group. Examples of the anteiso fatty acid include an anteiso fatty acid with 9 or more and 23 or less total carbons. From the viewpoint of being likelier to be controlled by the metabolism controlling method of the present invention, examples thereof include an anteiso fatty acid with 13 or more and 21 or less total carbons.

More specific examples of the anteiso fatty acid include one or more branched fatty acids selected from 6-methyloctanoic acid, 8-methyldecanoic acid, 12-methyltetradecanoic acid, 14-methylhexadecanoic acid, 16-methyloctadecanoic acid, 14-methylhexadecenoic acid, 16-methyloctadecenoic acid and salts thereof.

The branched fatty acid may be a salt, and examples of the salt include one or more salts selected from alkali metal salts, for example, a potassium salt and a sodium salt, and alkaline earth metal salts, for example, a magnesium salt and a calcium salt.

<Method for controlling metabolism of branched fatty acid>

The metabolism of branched fatty acids means the process of being converted into other compounds within microorganisms. The metabolism also includes a process in which branched fatty acids are incorporated into microorganisms and a process in which the incorporated branched fatty acids are metabolized for example by β oxidation to produce various kinds of compounds. For example, if an anteiso fatty acid with 17 carbons is incorporated into microorganisms and metabolized, the amount of branched fatty acids in systems is reduced, and conversion into other compounds occurs. If an anteiso fatty acid with 17 carbons is metabolized within microorganisms, 4-methyl-3-hexenoic acid (hereinafter also referred to as 4M3H), 4-methylhexanoic acid (hereinafter also referred to as 4MH), 2-methylbutyric acid (hereinafter also referred to as 2MBA) and the like, for example, are produced as metabolites. While 4M3H has the cis and trans forms as shown below, the present invention encompasses the compounds of both structures. 4M3H, 4MH and 2MBA are all known as substances causative of unpleasant odors.

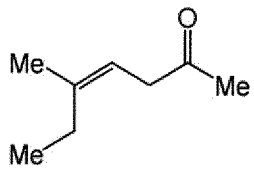
Cis form

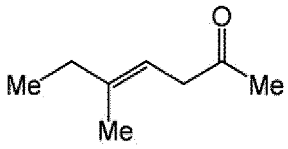
Trans form

In addition, if an iso fatty acid with 15 carbons is metabolized within microorganisms, isovaleric acid and the like, for example, are produced as metabolites.

Next, the metabolism controlling method of the present invention is explained.

The metabolism controlling method of the present invention includes bringing component (a) or components (a) and (b) into contact with a microorganism to control the metabolism of a branched fatty acid of the microorganism.

If components (a) and (b) are used, in the metabolism controlling method of the present invention, the mass ratio between the use amount of component (a) and the use amount of component (b), (b)/(a), is preferably 0.001 or more, more preferably 0.005 or more, further preferably 0.01 or more, furthermore preferably 0.03 or more and furthermore preferably 0.05 or more from the viewpoint of further raising the degree of control of metabolism in microorganisms, particularly from the viewpoint of further enhancing the rate of suppressing the metabolism of branched fatty acids or the viewpoint of further enhancing the metabolite production suppression rate for products of the metabolism of branched fatty acids, and preferably 1 or less, more preferably 0.5 or less, further preferably 0.3 or less and furthermore preferably 0.1 or less from the viewpoint of enabling further reduction in discharge loads to the environment.

The contact time of the microorganism and component (a) or components (a) and (b) can be appropriately determined depending on the degree of control of metabolism in microorganisms. From the viewpoint of further increasing the control of metabolism in microorganisms, the contact time is preferably a minute or more, more preferably 5 minutes or more, further preferably 10 minutes or more, furthermore preferably 30 minutes or more and furthermore preferably an hour or more, and preferably 72 hours or less, more preferably 48 hours or less and further preferably 24 hours or less. If components (a) and (b) are used, the present invention may be such that after bringing one of components (a) and (b) into contact with the microorganism, the other thereof is brought into contact with the microorganism, wherein a contact time within the above range preferably passes in the state where components (a) and (b) coexist.

In the metabolism controlling method of the present invention, the microorganism, component (a) and water are preferably brought into contact. In other words, the method for controlling the metabolism of a branched fatty acid of the present invention can be applied to a method in which component (a), water and a microorganism are brought into contact. The order in which they are brought into contact with one another is not limited. Examples of a method for bringing the microorganism, component (a) and water into contact include, for example,

(I) a method of bringing a composition including component (a) and water into contact with the microorganism,
(II) a method of bringing a mixture including water and the microorganism into contact with component (a),
(III) a method of bringing water into contact with a part where component (a) and the microorganism are present, and the like.

If components (a) and (b) are used, the microorganism, component (a), component (b) and water are preferably brought into contact in the metabolism controlling method of the present invention. In other words, the method for controlling the metabolism of a branched fatty acid of the present invention can be applied to a method in which component (a), component (b), water and a microorganism are brought into contact. The order in which they are brought into contact with one another is not limited. Examples of a method for bringing the microorganism, component (a), component (b) and water into contact include, for example,

(i) a method of bringing a composition including component (a), component (b) and water into contact with the microorganism,
(ii) a method of bringing a mixture including water and the microorganism into contact with a mixture including components (a) and (b),
(iii) a method of bringing water into contact with a part where component (a), component (b) and the microorganism are present,
(iv) a method of bringing a mixture including water and the microorganism into contact with components (a) and (b) separately or simultaneously,
(v) a method of bringing a composition including component (a) and water into contact with the microorganism and component (b) separately or simultaneously,
(vi) a method of bringing a composition including component (b) and water into contact with the microorganism and component (a) separately or simultaneously, and the like.

Examples of the above method (I) or (i) include, for example, a method of spraying or applying the composition including component (a) and water or the composition including component (a), component (b) and water on the microorganism, and a method of immersing the microorganism in the composition including component (a) and water or the composition including component (a), component (b) and water. Examples of the method of spraying the composition include, for example, a method of spraying it with an atomizer such as a sprayer or the like. Examples of the method of applying the composition include a method of applying the composition including component (a) and water or the composition including component (a), component (b) and water on the microorganism by bringing the microorganism into contact with an applicator, for example, a sheet-form tool or a roll-on type tool made of cotton fibers or synthetic fibers impregnated with the composition including component (a) and water or the composition including component (a), component (b) and water.

The contact of component (a) and water or component (a), component (b) and water with the microorganism may cause component (a), water and the microorganism or component (a), component (b), water and the microorganism to be in an intermixed state. The intermixed state of component (a), water and the microorganism or component (a), component (b), water and the microorganism may be a liquid state including component (a), water and the microorganism or component (a), component (b), water and the microorganism, and may be a state where such a liquid is present on the surface of a solid. The solid may be articles such as textile products, hard articles and the like, parts of the body such as skin, hair and the like, and others. The material of the textile products is not particularly limited and may be any of a natural material such as wool, silk, cotton or the like, a synthetic fiber such as polyester, polyamide or the like, and a combination thereof. In the present invention, the material of the textile products is preferably cotton. The textile products may be unused or already used once or more. The textile products may be damp or moist or sufficiently dried. The hard articles may be glass, metal, plastic or ceramic.

When the above method (II), (III) or (ii), (iii) is carried out on the surface of a solid, a method for allowing component (a) or components (a) and (b) to be present on the solid may be, for example, a method of allowing an optional component other than component (a) or components (a) and (b) to be present together with them on the surface of the solid in the range that the effects of component (a) or components (a) and (b) are not impaired. Specific examples thereof include an organic solvent and a surfactant other than component (a). Examples of the organic solvent include an organic solvent having a hydroxyl group, for example, a monovalent or more and hexavalent or less alcohol with 1 or more and 7 or less carbons. Examples of the surfactant include, for example, one or more surfactants selected from an anionic surfactant, a cationic surfactant, a nonionic surfactant [provided that component (a) is excluded] and an amphoteric surfactant.

In the metabolism controlling method of the present invention, component (a) or components (a) and (b) are preferably brought into contact with the microorganism under the presence of a branched fatty acid. The branched fatty acid is preferably those which can be metabolized by microorganisms. Examples of the branched fatty acid include, for example, the aforementioned branched fatty acids with 9 or more and 21 or less carbons. One or more fatty acids selected from an anteiso-type branched fatty acid and an iso-type branched fatty acid are also preferable as this branched fatty acid.

Examples of the metabolism controlling method including bringing component (a) into contact with the microorganism under the presence of a branched fatty acid include, for example,

(X1) a method of bringing the microorganism into contact with a place where the branched fatty acid and component (a) coexist,
(X2) a method of bringing the branched fatty acid into contact with a place where component (a) and the microorganism coexist,
(X3) a method of bringing component (a) into contact with a place where the branched fatty acid and the microorganism coexist, and the like. One or more methods selected from the above (X1) and (X2) are preferable and method (X1) is more preferable from the viewpoint of further controlling the metabolism of branched fatty acids, particularly from the viewpoint of further suppressing the metabolism of branched fatty acids.

Examples of the metabolism controlling method including bringing components (a) and (b) into contact with the microorganism under the presence of a branched fatty acid include, for example,

(x1) a method of bringing the microorganism into contact with a place where the branched fatty acid, component (a) and component (b) coexist,
(x2) a method of bringing the branched fatty acid into contact with a place where component (a), component (b) and the microorganism coexist,
(x3) a method of bringing components (a) and (b) into contact with a place where the branched fatty acid and the microorganism coexist, and the like. One or more methods selected from the above (x1) and (x2) are preferable and method (x1) is more preferable from the viewpoint of further controlling the metabolism of branched fatty acids, particularly from the viewpoint of further suppressing the metabolism of branched fatty acids.

The metabolism controlling method of the present invention is preferably a method for suppressing the metabolism of a branched fatty acid by a microorganism. Further, the metabolism controlling method of the present invention is preferably a method by which the amount of a branched fatty acid metabolized by a microorganism is suppressed while the viable bacterial count of the microorganism does not significantly change when component (a) or components (a) and (b) are brought into contact with the microorganism and when component (a) or components (a) and (b) are not brought into contact with the microorganism. In the present invention, the viable bacterial count of the microorganism is preferably substantially maintained before and after the contact of component (a). In the present invention, the viable bacterial count of the microorganism "being substantially maintained" may be, for example, a difference between logarithm value (1) of the viable bacterial count of the microorganism when component (a) and the microorganism are not brought into contact and logarithm value (2) of the viable bacterial count of the microorganism when component (a) and the microorganism are brought into contact, logarithm value (1)-logarithm value (2), being - 1 or more and less than 2. The difference between the logarithm values may be generally called bacterial activity value or bacteriostatic activity value, and in JIS L 1902:2015, for example, having bactericidal or antibacterial properties is defined as having a bactericidal or bacteriostatic activity value of 2.2 or more in a test for bactericidal or antibacterial properties. In the present invention, excessive bactericidal activity by component (a) is not preferable, and the viable bacterial count of the microorganism may be considered to be "substantially maintained" when the bactericidal or bacteriostatic activity value is, for example, -1 or more and less than 2, preferably -0.5 or more and less than 1, more preferably 0 or more and 0.8 or less, and further preferably 0 or more and 0.7 or less. Note that, when components (a) and (b) are used in the present invention, the fact that the viable bacterial count of the microorganism is "substantially maintained" can be judged by a difference based on logarithm values of the viable bacterial count measured by using "components (a) and (b)" instead of "component (a)."

Examples of the method for controlling the metabolism of a branched fatty acid of the present invention include a method for controlling the metabolism of a branched fatty acid including, bringing a treatment liquid containing component (a) or components (a) and (b), a branched fatty acid with 9 or more and 21 or less carbons and an organic solvent into contact with a microorganism to control the metabolism of a branched fatty acid of the microorganism.

The microorganism is preferably one or more microorganisms selected from bacteria of the genus Moraxella, bacteria of the genus Micrococcus, bacteria of the genus Escherichia, bacteria of the genus Staphylococcus, bacteria of the genus Corynebacterium and yeast of the genus Rhodotorula.

Component (a) is preferably a compound in which n in the general formula (1) is 0.

The content of component (a) in the treatment liquid is preferably 10 ppm or more and 10 mass% or less.

When component (b) is used, the content of component (b) in the treatment liquid is preferably 0.01 ppm or more and 10 ppm or less.

Component (b) used in the treatment liquid is preferably one or more compounds selected from the following components (b1), (b2), (b3) and (b4), further one or more compounds selected from the following components (b1) and (b3), further one or more compounds selected from the following component (b1), and further one or more compounds selected from 4-4’-dichloro-2-hydroxydiphenyl ether (diclosan), 2,4,4’-trichloro-2’-hydroxydiphenyl ether (triclosan) and cerulenin,

component (b1): a compound having a diphenyl ether group,
component (b2): thiolactomycin or a thiolactomycin derivative,
component (b3): cerulenin or a cerulenin derivative, and
component (b4): α-methylene-γ-butyrolactone or an α-methylene-γ-butyrolactone derivative.

The content of the above branched fatty acid in the treatment liquid is preferably 1 ppm or more and 1 mass% or less.

The balance of the treatment liquid is preferably an organic solvent. The organic solvent is preferably methanol.

The contact time of the treatment liquid is preferably a minute or more and 64 hours or less. The effects of the present invention can be obtained while the above component (a) or components (a) and (b) are brought into contact with the microorganism. The contact time can be appropriately changed depending on the technical field to which the present invention is applied.

The contact temperature of the treatment liquid is preferably 1°C or more and 45°C or less, and preferably 5°C or more and more preferably 10°C or more from the viewpoint of being likelier to obtain the effects of the present invention, and preferably 40°C or less and more preferably 35°C or less from the same viewpoint.

The treatment liquid may be brought into contact with a mixture including the microorganism and water.

The present invention provides an agent for controlling the metabolism of a branched fatty acid in a microorganism containing component (a). In addition, the present invention provides an agent for controlling the metabolism of a branched fatty acid in a microorganism containing components (a) and (b). The metabolism controlling agent of the present invention contains component (a) or components (a) and (b) as active ingredients for controlling the metabolism of a branched fatty acid in a microorganism. The matters mentioned in the method for controlling the metabolism of a branched fatty acid of the present invention can be appropriately applied to the metabolism controlling agent of the present invention. For example, specific examples and preferable modes of components (a) and (b) and the like are also the same as those in the method for controlling the metabolism of a branched fatty acid of the present invention. In the agent for controlling the metabolism of a branched fatty acid containing components (a) and (b) of the present invention, the mass ratio between the content of component (a) and the content of component (b), (b)/(a), is preferably 0.001 or more, more preferably 0.005 or more, further preferably 0.01 or more, furthermore preferably 0.03 or more and furthermore preferably 0.05 or more from the viewpoint of further raising the degree of control of metabolism in microorganisms, particularly from the viewpoint of further enhancing the rate of suppressing the metabolism of branched fatty acids or the viewpoint of further enhancing the metabolite production suppression rate for products of the metabolism of branched fatty acids, and preferably 1 or less, more preferably 0.5 or less, further preferably 0.3 or less and furthermore preferably 0.1 or less from the viewpoint of enabling further reduction in discharge loads to the environment.

The agent for controlling the metabolism of a branched fatty acid of the present invention may be an agent composed of component (a) or components (a) and (b). In addition, the agent for controlling the metabolism of a branched fatty acid of the present invention may be used in combination with a further optional component [hereinafter referred to as component (c)]. Examples of component (c) include (c1) a solvent and (c2) a surfactant other than component (a). Examples of solvent (c1) include one or more compounds selected from water and an organic solvent. The water may be ion exchange water, distilled water, tap water, or water including hardness components such as calcium, magnesium or the like. The organic solvent is preferably an organic solvent having a hydroxyl group from the viewpoint of further enhancing the effect of controlling the metabolism of branched fatty acids of the present invention. Examples of the organic solvent having a hydroxyl group include a monovalent or more and hexavalent or less alcohol with 1 or more and 7 or less carbons. Specific examples thereof include monovalent alcohols with 1 or more and 7 or less carbons such as, for example, methanol, ethanol and isopropanol. In addition, divalent alcohols with 2 or more 7 or less carbons such as, for example, ethylene glycol, propylene glycol and hexylene glycol are included. Further, trivalent alcohols with 3 or more and 7 or less carbons such as, for example, glycerol and 1,2,3-propanetriol are included. Tetravalent or more and hexavalent or less alcohols with 5 or more and 7 or less carbons such as, for example, pentaerythritol and sugar alcohol are included. In addition, examples of surfactant (c2) include one or more surfactants selected from an anionic surfactant, a nonionic surfactant, an amphoteric surfactant and a cationic surfactant.

The present invention provides the use of component (a) or components (a) and (b) as an agent for controlling the metabolism of a branched fatty acid in a microorganism. The use as a metabolism controlling agent of the present invention is to use component (a) or components (a) and (b) for the purpose of controlling the metabolism of a branched fatty acid in a microorganism. The matters mentioned in the method for controlling the metabolism of a branched fatty acid of the present invention can be appropriately applied to the use of component (a) or components (a) and (b) as an agent for controlling the metabolism of a branched fatty acid in a microorganism of the present invention. For example, specific examples and preferable modes of components (a) and (b) and the like are also the same as those in the method for controlling the metabolism of a branched fatty acid of the present invention.

The present invention discloses, in addition to the embodiments mentioned above, the following aspects.

<1> A method for controlling the metabolism of a branched fatty acid including, bringing (a) one or more compounds selected from a compound represented by the following general formula (1) [hereinafter referred to as component (a)] into contact with a microorganism to control the metabolism of a branched fatty acid of the microorganism,

$$RO\text{-}(C_2H_4O)_n\text{-}H \qquad (1)$$

wherein R is a linear aliphatic hydrocarbon group with 9 or more and 16 or less carbons, and n is 0 or 1.
<2> The method for controlling the metabolism of a branched fatty acid according to <1>, wherein the contact time of the microorganism and component (a) is a minute or more, preferably 5 minutes or more, more preferably 10 minutes or more, further preferably 30 minutes or more and furthermore preferably an hour or more, and 72 hours or less, preferably 48 hours or less and more preferably 24 hours or less.
<3> The method for controlling the metabolism of a branched fatty acid according to <1> or <2>, wherein the microorganism, component (a) and water are brought into contact.
<4> The method for controlling the metabolism of a branched fatty acid according to <3>, wherein a method for bringing the microorganism, component (a) and water into contact is any of the following (I) to (III):

(I) a method of bringing a composition including component (a) and water into contact with the microorganism;
(II) a method of bringing a mixture including water and the microorganism into contact with component (a); and
(III) a method of bringing water into contact with a part where component (a) and the microorganism are present.

<5> The method for controlling the metabolism of a branched fatty acid according to any of <1> to <4>, wherein component (a) is brought into contact with the microorganism under the presence of a branched fatty acid.
<6> The method for controlling the metabolism of a branched fatty acid according to <5>, wherein a method for bringing component (a) into contact with the microorganism under the presence of a branched fatty acid is one or more methods selected from the following (X1) and (X2):

(X1) a method of bringing the microorganism into contact with a place where the branched fatty acid and component (a) coexist;
(X2) a method of bringing the branched fatty acid into contact with a place where component (a) and the microorganism coexist; and
(X3) a method of bringing component (a) into contact with a place where the branched fatty acid and the microorganism coexist.

<7> The method for controlling the metabolism of a branched fatty acid according to any of <1> to <6>, wherein the viable bacterial count of the microorganism is substantially maintained before and after the contact of component (a).
<8> The method for controlling the metabolism of a branched fatty acid according to <7>, wherein "being substantially maintained" is defined as a difference between logarithm value (1) of the viable bacterial count of the microorganism when component (a) and the microorganism are not brought into contact and logarithm value (2) of the viable bacterial count of the microorganism when component (a) and the microorganism are brought into contact, logarithm value (1)-logarithm value (2), being -1 or more and less than 2.
<9> The method for controlling the metabolism of a branched fatty acid according to <7>, wherein "being substantially maintained" is defined as the bactericidal or bacteriostatic activity value in JIS L 1902:2015 determined by using logarithm value (1) of the viable bacterial count of the microorganism when component (a) and the microorganism are not brought into contact and logarithm value (2) of the viable bacterial count of the microorganism when component (a) and the microorganism are brought into contact being -1 or more and less than 2, preferably -0.5 or more and less than 1, more preferably 0 or more and 0.8 or less and further preferably 0 or more and 0.7 or less.
<10> The method for controlling the metabolism of a branched fatty acid according to any of <1> to <9>, wherein component (a) and (b) an inhibiting agent for fatty acid synthases are brought into contact with the microorganism.
<11> A method for controlling the metabolism of a branched fatty acid including, bringing (a) one or more compounds selected from a compound represented by the following general formula (1) [hereinafter referred to as component (a)] and (b) an inhibiting agent for fatty acid synthases [hereinafter referred to as component (b)] into contact with a microorganism to control the metabolism of a branched fatty acid of the microorganism,

$$RO\text{-}(C_2H_4O)_n\text{-}H \qquad (1)$$

wherein R is a linear aliphatic hydrocarbon group with 9 or more and 16 or less carbons, and n is 0 or 1.
<12> The method for controlling the metabolism of a branched fatty acid according to <10> or <11>, wherein component (b) is one or more compounds selected from the following components (b1), (b2), (b3) and (b4):

component (b1): a compound having a diphenyl ether group;
component (b2): thiolactomycin or a thiolactomycin derivative;
component (b3): cerulenin or a cerulenin derivative; and
component (b4): α-methylene-γ-butyrolactone or an α-methylene-γ-butyrolactone derivative.

<13> The method for controlling the metabolism of a branched fatty acid according to <12>, wherein component (b1) is a compound in which one or more hydrogen atoms of a phenyl group are substituted for by a hydroxyl group, and preferably a compound in which two or more hydrogen atoms of a phenyl group are substituted for by a hydroxyl group and a chlorine group.
<14> The method for controlling the metabolism of a branched fatty acid according to <12> or <13>, wherein component (b1) is one or more compounds selected from 4-4'-dichloro-2-hydroxydiphenyl ether (diclosan) and 2,4,4'-trichloro-2'-hydroxydiphenyl ether (triclosan).
<15> The method for controlling the metabolism of a branched fatty acid according to any of <10> to <14>, wherein the microorganism, component (a), component (b) and water are brought into contact.
<16> The method for controlling the metabolism of a branched fatty acid according to <15>, wherein a method for bringing the microorganism, component (a), component (b) and water into contact is any of the following (i) to (vi) :

(i) a method of bringing a composition including component (a), component (b) and water into contact with the microorganism;
(ii) a method of bringing a mixture including water and the microorganism into contact with a mixture including components (a) and (b);
(iii) a method of bringing water into contact with a part where component (a), component (b) and the microorganism are present;
(iv) a method of bringing a mixture including water and the microorganism into contact with components (a) and (b) separately or simultaneously;
(v) a method of bringing a composition including component (a) and water into contact with the microorganism and component (b) separately or simultaneously; and
(vi) a method of bringing a composition including component (b) and water into contact with the microorganism and component (a) separately or simultaneously.

<17> The method for controlling the metabolism of a branched fatty acid according to any of <10> to <16>, wherein components (a) and (b) are brought into contact with the microorganism under the presence of a branched fatty acid.
<18> The method for controlling the metabolism of a branched fatty acid according to <17>, wherein a method for bringing components (a) and (b) into contact with the microorganism under the presence of a branched fatty acid is a method selected from the following (x1) to (x3):

(x1) a method of bringing the microorganism into contact with a place where the branched fatty acid, component (a) and component (b) coexist;
(x2) a method of bringing the branched fatty acid into contact with a place where component (a), component (b) and the microorganism coexist; and
(x3) a method of bringing components (a) and (b) into contact with a place where the branched fatty acid and the microorganism coexist.

<19> The method for controlling the metabolism of a branched fatty acid according to any of <10> to <18>, wherein the viable bacterial count of the microorganism is substantially maintained before and after the contact of components (a) and (b).
<20> The method for controlling the metabolism of a branched fatty acid according to <19>, wherein "being substantially maintained" is defined as a difference between logarithm value (1) of the viable bacterial count of the microorganism when component (a), component (b) and the microorganism are not brought into contact and logarithm value (2) of the viable bacterial count of the microorganism when component (a), component (b) and the microorganism are brought into contact, logarithm value (1)-logarithm value (2), being -1 or more and less than 2.
<21> The method for controlling the metabolism of a branched fatty acid according to <19>, wherein "being substantially maintained" is defined as the bactericidal or bacteriostatic activity value in JIS L 1902:2015 determined by using logarithm value (1) of the viable bacterial count of the microorganism when component (a), component (b) and the microorganism are not brought into contact and logarithm value (2) of the viable bacterial count of the microorganism when component (a), component (b) and the microorganism are brought into contact being -1 or more and less than 2, preferably -0.5 or more and less than 1, more preferably 0 or more and 0.8 or less and further preferably 0 or more and 0.7 or less.

<22> The method for controlling the metabolism of a branched fatty acid according to any of <10> to <21>, wherein the mass ratio between the use amount of component (a) and the use amount of component (b), (b)/(a), is preferably 0.001 or more, more preferably 0.005 or more, further preferably 0.01 or more, furthermore preferably 0.03 or more and furthermore preferably 0.05 or more, and preferably 1 or less, more preferably 0.5 or less, further preferably 0.3 or less and furthermore preferably 0.1 or less.
<23> The method for controlling the metabolism of a branched fatty acid according to any of <1> to <22>, wherein R in the general formula (1) has 10 or more, preferably 11 or more and more preferably 12 or more, and 15 or less and preferably 14 or less carbons.
<24> The method for controlling the metabolism of a branched fatty acid according to any of <1> to <23>, wherein the proportion of a compound in which R has 12 or 13 carbons in component (a) is 30 mass% or more, preferably 50 mass% or more, more preferably 60 mass% or more, further preferably 70 mass% or more, furthermore preferably 80 mass% or more, furthermore preferably 90 mass% or more, furthermore preferably 95 mass% or more and furthermore preferably 97 mass% or more, and 100 mass% or less, or 100 mass%.
<25> The method for controlling the metabolism of a branched fatty acid according to any of <1> to <24>, wherein a compound in which n in the general formula (1) is 0 is one or more compounds selected from 1-nonyl alcohol, 1-decanol, 1-undecanol, 1-dodecanol, 1-tridecanol, 1-tetradecanol, 1-pentadecanol and 1-hexadecanol.
<26> The method for controlling the metabolism of a branched fatty acid according to any of <1> to <25>, wherein a compound in which n in the general formula (1) is 1 is one or more compounds selected from ethylene glycol mono-1-dodecyl ether, ethylene glycol mono-1-tridecyl ether and ethylene glycol mono-1-tetradecyl ether.
<27> The method for controlling the metabolism of a branched fatty acid according to any of <1> to <26>, wherein the microorganism is one or more microorganisms selected from gram-negative bacteria, gram-positive bacteria and yeast.
<28> The method for controlling the metabolism of a branched fatty acid according to <27>, wherein the gram-negative bacteria are gram-negative bacteria selected from bacteria of the genus Moraxella, bacteria of the genus Acinetobacter, bacteria of the genus Pseudomonas, bacteria of the genus Sphingomonas, bacteria of the genus Ralstonia, bacteria of the genus Cupriavidus, bacteria of the genus Psychrobacter, bacteria of the genus Serratia, bacteria of the genus Escherichia and bacteria of the genus Burkholderia.
<29> The method for controlling the metabolism of a branched fatty acid according to <27> or <28>, wherein the gram-positive bacteria are gram-positive bacteria selected from bacteria of the genus Bacillus, bacteria of the genus Staphylococcus, bacteria of the genus Micrococcus and bacteria of the genus Corynebacterium.
<30> The method for controlling the metabolism of a branched fatty acid according to any of <1> to <29>, wherein the branched fatty acid is a branched fatty acid with 9 or more and 21 or less carbons.
<31> The method for controlling the metabolism of a branched fatty acid according to any of <1> to <30>, wherein the branched fatty acid is a saturated or unsaturated branched fatty acid with 13 or more and 19 or less carbons, and preferably a saturated or unsaturated branched fatty acid with 15 or more and 19 or less carbons.
<32> The method for controlling the metabolism of a branched fatty acid according to any of <1> to <31>, wherein the branched fatty acid is one or more branched fatty acids selected from a 10-methyl fatty acid, an iso fatty acid and an anteiso fatty acid or a branched fatty acid having one or more methyl groups as branched chains.
<33> The method for controlling the metabolism of a branched fatty acid according to <32>, wherein the 10-methyl fatty acid is a 10-methyl fatty acid with 15 or more and 18 or less total carbons, and preferably a branched fatty acid selected from 10-methylpentadecanoic acid, 10-methylhexadecanoic acid, 1-methylheptadecanoic acid and 10-methyloctadecanoic acid.
<34> The method for controlling the metabolism of a branched fatty acid according to <32> or <33>, wherein the iso fatty acid, which is a fatty acid in which a methyl branch is attached to one carbon before the $\omega$ end of the fatty acid, or the carbon at $\omega$-1, is preferably an iso fatty acid with 10 or more and 24 or less total carbons, and more preferably an iso fatty acid with 12 or more and 18 or less total carbons.
<35> The method for controlling the metabolism of a branched fatty acid according to any of <32> to <34>, wherein the anteiso fatty acid, which is an anteiso-type branched fatty acid in which a methyl group substitutes for the third carbon counting from the terminal methyl group, is preferably an anteiso fatty acid with 9 or more and 23 or less total carbons, more preferably an anteiso fatty acid with 13 or more and 21 or less total carbons, and further preferably one or more branched fatty acids selected from 6-methyloctanoic acid, 8-methyldecanoic acid, 12-methyltetradecanoic acid, 14-methylhexadecanoic acid, 16-methyloctadecanoic acid, 14-methylhexadecenoic acid, 16-methyloctadecenoic acid and salts thereof.
<36> The method for controlling the metabolism of a branched fatty acid according to any of <1> to <35>, wherein the metabolism of the branched fatty acid of the microorganism is $\beta$ oxidization.
<37> An agent for controlling the metabolism of a branched fatty acid in a microorganism containing, (a) one or more compounds selected from a compound represented by the following general formula (1),

$$RO\text{-}(C_2H_4O)_n\text{-}H \qquad (1)$$

wherein R is a linear aliphatic hydrocarbon group with 9 or more and 16 or less carbons, and n is 0 or 1.

<38> An agent for controlling the metabolism of a branched fatty acid in a microorganism containing, (a) one or more compounds selected from a compound represented by the following general formula (1) and (b) an inhibiting agent for fatty acid synthases,

$$RO\text{-}(C_2H_4O)_n\text{-}H \qquad (1)$$

wherein R is a linear aliphatic hydrocarbon group with 9 or more and 16 or less carbons, and n is 0 or 1.

<39> The agent for controlling the metabolism of a branched fatty acid according to <38>, wherein the mass ratio between the content of component (a) and the content of component (b), (b)/(a), is preferably 0.001 or more, more preferably 0.005 or more, further preferably 0.01 or more, furthermore preferably 0.03 or more and furthermore preferably 0.05 or more, and preferably 1 or less, more preferably 0.5 or less, further preferably 0.3 or less and furthermore preferably 0.1 or less.

<40> The agent for controlling the metabolism of a branched fatty acid according to any of <37> to <39>, wherein the metabolism of the branched fatty acid of the microorganism is β oxidization.

<41> Use as an agent for controlling the metabolism of a branched fatty acid in a microorganism of (a) one or more compounds selected from a compound represented by the following general formula (1),

$$RO\text{-}(C_2H_4O)_n\text{-}H \qquad (1)$$

wherein R is a linear aliphatic hydrocarbon group with 9 or more and 16 or less carbons, and n is 0 or 1.

<42> Use as an agent for controlling the metabolism of a branched fatty acid in a microorganism of a composition containing (a) one or more compounds selected from a compound represented by the following general formula (1) and (b) an inhibiting agent for fatty acid synthases,

$$RO\text{-}(C_2H_4O)_n\text{-}H \qquad (1)$$

wherein R is a linear aliphatic hydrocarbon group with 9 or more and 16 or less carbons, and n is 0 or 1.

## Examples

The compounds used in the examples and comparative examples are listed below.

[Metabolism controlling agent]

<Component (a) >

C9OH: 1-nonyl alcohol
C10OH: 1-decanol
C11OH: 1-undecanol
C12OH: 1-dodecanol
C13OH: 1-tridecanol
C14OH: 1-tetradecanol
C15OH: 1-pentadecanol
C16OH: 1-hexadecanol
C12E1: ethylene glycol mono-1-dodecyl ether (a compound of the general formula (1) in which R is a dodecyl group and n is 1)

<Component (a'): comparative compound for component (a)>

C18OH: 1-octadecanol
C12E2: diethylene glycol mono-1-dodecyl ether (a compound of the general formula (1) in which R is a 1-dodecyl

group and n is 2)
C12E5: pentaethylene glycol mono-1-dodecyl ether (a compound of the general formula (1) in which R is a dodecyl group and n is 5)
4-C12OH: 4-methyl-1-undecanol

[Branched fatty acid]

14-methylhexadecanoic acid: manufactured by Sigma-Aldrich Co. LLC
13-methyltetradecanoic acid: manufactured by Larodan Fine Chemicals

<Example 1 and comparative example 1>

The suppression of metabolism of 14-methylhexadecanoic acid by bacteria of the genus Moraxella and the suppression of a metabolite therefrom were tested in the following manner. The results are shown in Table 1.

(1) Suppression of metabolism of 14-methylhexadecanoic acid

Moraxella osloensis (strain isolated from clothing) was precultured on an SCD-LP agar medium (NIHON PHARMACEUTICAL CO., LTD.) at 37°C for 24 hours, and a bacterial suspension of $10^6$ (CFU/ml) was prepared by using an NB liquid medium (Difco) diluted to 1/20 with ion exchange water.
A methanol solution in which 14-methylhexadecanoic acid (SIGMA-ALDRICH) was dissolved was added dropwise to 3 cmx3 cm pieces of sterilized plain-weave cotton fabric to make it adhered to the fabric. The adhesion amount of 14-methylhexadecanoic acid was set to 10 μg per piece of the plain-weave cotton fabric (100 μg/fabric g). Further, a methanol solution in which each metabolism controlling agent was dissolved was prepared and added dropwise to the fabric to which 14-methylhexadecanoic acid adhered, and it was thereafter dried for an hour. The methanol solution was added dropwise such that the amount of the metabolism controlling agent was as shown in the table. This fabric was placed in a sterilized screw tube bottle No. 3 (Maruemu Corporation) and 100 μl of the bacterial suspension was inoculated thereto, and culture was carried out under 37°C conditions for 21 hours.
3 ml of methanol was added to the screw tube bottle after culture, and extraction was carried out under ultrasonic irradiation for 30 minutes (20°C). The extraction liquid was mixed with a (trimethylsilyl)diazomethane hexane solution (FUJIFILM Wako Pure Chemical Corporation) at 9:1 (volume ratio) and left overnight under room temperature and dark place conditions. After that, 14-methylhexadecanoic acid remaining on the fabric was quantified by GC. The quantification conditions for GC are as follows.

- GC: Agilent 7890A
- GC column: Agilent DB-1 30 mx250 μm×0.25 μm
- Temperature rise condition: 50°C (held for 3 minutes)-(10°C/min)-300°C (held for 5 minutes)
- Sample injection volume: 1 μl
- Split ratio: 1:12
- Detector: FID

The residual amount of 14-methylhexadecanoic acid after culture on the fabric to which the metabolism controlling agent was added was quantified, and the ratio of the residual amount to that of 14-methylhexadecanoic acid in the case of adding no metabolism controlling agent (control) was calculated as a metabolism suppression rate (%). The higher a numerical value thereof is, the more the metabolism is suppressed. Note that the metabolism suppression rate was determined by the following formula:

$$\text{Metabolism suppression rate (\%)} = [1-(100-A)/(100-B)]\times 100$$

A: residual amount of 14-methylhexadecanoic acid of example or comparative example (μg/fabric g)
B: residual amount of 14-methylhexadecanoic acid of control (μg/fabric g)

(2) Suppression of metabolite

After culture on fabric prepared in the same manner as in the above (1), 3 ml of methanol was added, and extraction was carried out under ultrasonic irradiation for 30 minutes (20°C). The extraction liquid was mixed with (a 1000 ppm methanol solution of) an ADAM reagent of Funakoshi Co., Ltd. at 1:1 (volume ratio) and left overnight under room temperature and dark place conditions. After that, 4-methyl-3-hexenoic acid as a metabolite from 14-methylhexadecanoic acid was quantified by HPLC. The quantification conditions are as follows.

Quantification conditions for metabolite

LC: HITACHI ELITE LaChrom
Column: Zorbax C8 4.6×250 mm
Eluent: acetonitrile 61% (v/v), water 39% (v/v)
Column temperature: 40°C
Sample injection volume: 10 μL
Flow rate: 1.0 mL/min
Detection: FLD Ex. 365 nm, Em. 412 nm

The production amount of 4-methyl-3-hexenoic acid after culture on the fabric to which each metabolism controlling agent was added was quantified, and the ratio of the production amount to that of 4-methyl-3-hexenoic acid in the case of adding no metabolism controlling agent (control) was calculated as a metabolite suppression rate (%). The higher a numerical value thereof is, the more the metabolism is suppressed. Note that the metabolite suppression rate was determined by the following formula:

$$\text{Metabolite suppression rate (\%)} = 100 \times [(D-C)/D]$$

C: production amount of metabolite of example or comparative example (μg/fabric g)
D: production amount of metabolite of control (μg/fabric g)

(3) Measurement of viable bacterial count

After culture on fabric prepared in the same manner as in the above (1), 9 mL of an LP diluent (manufactured by NIHON PHARMACEUTICAL CO., LTD.) was added, and the extraction of the bacteria was carried out under ultrasound for 10 minutes. The extraction liquid was poured and mixed to an SCD-LP agar medium (NIHON PHARMACEUTICAL CO., LTD.), and culture was thereafter carried out at 37°C for a day. The number of colonies obtained on each piece of the fabric was counted, and a common logarithm value thereof was measured as the viable bacterial count. A difference between logarithm value (1) of the viable bacterial count of the microorganism when no metabolism controlling agent is added and logarithm value (2) of the viable bacterial count of the microorganism when a metabolism controlling agent is added, logarithm value (1)-logarithm value (2), being -1 or more and less than 2 means that the viable bacterial count does not change regardless of the presence or absence of the contact of a metabolism controlling agent and the viable bacterial count of the microorganism is maintained. In addition, the closer a value thereof is to 0, the more the viable bacterial count of the microorganism is maintained.

[Table 1]

| <Microorganism:*Moraxella osloensis*> | | | | | | | | | | | | | | | |
|---|---|---|---|---|---|---|---|---|---|---|---|---|---|---|---|
| | | | Example | | | | | | | | | Comparative example | | | Control |
| | | | 1-1 | 1-2 | 1-3 | 1-4 | 1-5 | 1-6 | 1-7 | 1-8 | 1-9 | 1-1 | 1-2 | 1-3 | |
| Metabolism controlling agent | | | C9OH | C10OH | C11OH | C12OH | C13OH | C14OH | C15OH | C16OH | C12E1 | C18OH | C12E5 | 4-C12OH | |
| Metabolism suppression rate (%) | Amount of metabolism controlling agent **(µg/ fabric g)** | 100 | - | - | 14 | 42 | 48 | 30 | 21 | 12 | 29 | 2 | 2 | -10 | 0 |
| | | 250 | 13 | 18 | 24 | 91 | 93 | 67 | 84 | 37 | 90 | 9 | 4 | 8 | 0 |
| Metabolite suppression rate (%) | Amount of metabolism controlling agent **(µg/ fabric g)** | 100 | - | - | 53 | 70 | 87 | 57 | 69 | 45 | 63 | 2 | 8 | -23 | 0 |
| | | 250 | 18 | 14 | 95 | 87 | 94 | 93 | 97 | 72 | 70 | 12 | 2 | 0 | 0 |
| Bacterial count (Log (cfu/fabric $cm^2$)) | Amount of metabolism controlling agent **(µg/ fabric g)** | 100 | - | - | 5.8 | 6.2 | 6.2 | 5.9 | 6.0 | 6.1 | 6.1 | 5.7 | 6.3 | 6.6 | 6.0 |
| | | 250 | 6.2 | 5.9 | 6.3 | 6.4 | 6.1 | 6.1 | 5.9 | 6.2 | 6.0 | 6.3 | 6.1 | 5.0 | 6.0 |

<Example 2 and comparative example 2>

The metabolism of 14-methylhexadecanoic acid by bacteria of the genus Micrococcus was tested in the same manner as in example 1. The results are shown in Table 2.

In this example, Micrococcus aloeverae (strain isolated from clothing) was used as a microorganism.

Further, in this example, a bacterial suspension of $10^7$ (CFU/ml) prepared by using an SCD liquid medium (NIHON PHARMACEUTICAL CO., LTD.) diluted to 1/10 with ion exchange water was used as a bacterial suspension.

Further, in this example, 2-methylbutanoic acid was quantified as a metabolite from 14-methylhexadecanoic acid, and the metabolite suppression rate was determined in the same manner as in example 1.

[Table 2]

| <Microorganism:*Micrococcus aloeverae* > | | | | | | | | | |
|---|---|---|---|---|---|---|---|---|---|
| | | | Example | | | Comparative example | | | Control |
| | | | 2-1 | 2-2 | 2-3 | 2-1 | 2-2 | 2-3 | |
| Metabolism controlling agent | | | C10OH | C12OH | C13OH | C18OH | C12E2 | C12E5 | |
| Metabolism suppression rate (%) | Amount of metabolism controlling agent **(μg/ fabric g)** | 250 | 33 | 73 | 41 | -7 | -11 | -3 | 0 |
| Metabolite suppression rate (%) | Amount of metabolism controlling agent **(μg/ fabric g)** | 250 | 51 | 79 | 59 | 1 | 8 | 6 | 0 |
| Bacterial count (Log (cfu/fabric $cm^2$)) | Amount of metabolism controlling agent **(μg/ fabric g)** | 250 | 7.0 | 6.9 | 6.7 | 6.8 | 6.8 | 6.6 | 6.9 |

<Example 3 and comparative example 3>

The metabolism of 14-methylhexadecanoic acid by bacteria of the genus Staphylococcus (Staphylococcus aureus) was tested in the same manner as in example 1. The results are shown in Table 3.

In this example, Staphylococcus aureus (strain isolated from clothing) was used as a microorganism.

Further, in this example, a bacterial suspension of $10^7$ (CFU/ml) prepared by using an NB liquid medium diluted to 1/10 with ion exchange water was used as a bacterial suspension.

Further, in this example, 4-methylhexanoic acid was quantified as a metabolite from 14-methylhexadecanoic acid, and the metabolite suppression rate was determined in the same manner as in example 1.

[Table 3]

| <Microorganism: *Staphylococcus aureus* > | | | | | | | | | |
|---|---|---|---|---|---|---|---|---|---|
| | | | Example | | | | Comparative example | | Control |
| | | | 3-1 | 3-2 | 3-3 | 3-4 | 3-1 | 3-2 | |
| Metabolism controlling agent | | | C10OH | C12OH | C14OH | C16OH | C18OH | C12E2 | |
| Metabolism suppression rate (%) | Amount of metabolism controlling agent **(μg/ fabric g)** | 250 | 16 | 75 | 42 | 16 | -1 | 7 | 0 |

(continued)

| <Microorganism: *Staphylococcus aureus* > | | | | | | | | | |
|---|---|---|---|---|---|---|---|---|---|
| | | | Example | | | | Comparative example | | Control |
| | | | 3-1 | 3-2 | 3-3 | 3-4 | 3-1 | 3-2 | |
| Metabolism controlling agent | | | C10OH | C12OH | C14OH | C16OH | C18OH | C12E2 | |
| Metabolite suppression rate (%) | Amount of metabolism controlling agent **(μg/ fabric g)** | 250 | 35 | 74 | 53 | 20 | - | - | 0 |
| Bacterial count (Log (cfu/fabric $cm^2$)) | Amount of metabolism controlling agent **(μg/ fabric g)** | 250 | 5.5 | 5.7 | 5.6 | 6.1 | 5.6 | - | 5.5 |

<Example 4 and comparative example 4>

The metabolism of 14-methylhexadecanoic acid by bacteria of the genus Escherichia (Escherichia coli) was tested in the same manner as in example 1. The results are shown in Table 4.

In this example, Escherichia coli (NBRC3972) was used as a microorganism.

Further, in this example, a bacterial suspension of $10^6$ (CFU/ml) prepared by using an NB liquid medium diluted to 1/10 with ion exchange water was used as a bacterial suspension.

Further, in this example, 4-methylhexanoic acid was quantified as a metabolite from 14-methylhexadecanoic acid, and the metabolite suppression rate was determined in the same manner as in example 1.

[Table 4]

| <Microorganism:*Escherichia coli*> | | | | | | | | | | |
|---|---|---|---|---|---|---|---|---|---|---|
| | | | Example | | | | | Comparative example | | Control |
| | | | 4-1 | 4-2 | 4-3 | 4-4 | 4-5 | 4-1 | 4-2 | |
| Metabolism controlling agent | | | C9OH | C10OH | C12OH | C14OH | C16OH | C18OH | C12E2 | |
| Metabolism suppression rate (%) | Amount of metabolism controlling agent **(μg/fabric g)** | 500 | 13 | 16 | 39 | 27 | - | -15 | -2 | 0 |
| | | 1000 | 25 | 38 | 90 | 67 | 18 | 3 | - | 0 |
| Metabolite suppression rate (%) | Amount of metabolism controlling agent **(μg/fabric g)** | 500 | 31 | 42 | 74 | 47 | - | - | -2 | 0 |
| | | 1000 | 33 | 75 | 93 | 77 | 27 | 0 | - | 0 |
| Bacterial count (Log (cfu/ fabric $cm^2$)) | Amount of metabolism controlling agent **(μg/fabric g)** | 500 | 6.5 | 6.5 | 6.3 | 6.7 | - | 6.2 | 6.5 | 6.4 |
| | | 1000 | 6.3 | 6.4 | 6.3 | 6.5 | 6.6 | 5.9 | - | 6.4 |

<Example 5 and comparative example 5>

The metabolism of 14-methylhexadecanoic acid by yeast of the genus Rhodotorula was tested in the same manner as in example 1. The results are shown in Table 5.

In this example, Rhodotorula mucilaginosa (strain isolated from clothing) was used as a microorganism.

Further, in this example, the bacteria were precultured under conditions of a PDA agar medium (Difco), 30°C and 48 hours.

Further, in this example, a bacterial suspension of $10^7$ (CFU/ml) prepared by using a PDB liquid medium (Difco) diluted to 1/10 with ion exchange water was used as a bacterial suspension.

Further, in this example, 4-methylhexanoic acid was quantified as a metabolite from 14-methylhexadecanoic acid, and the metabolite suppression rate was determined in the same manner as in example 1.

Further, in this example, after the extraction of the bacteria in (3) of example 1, they were poured and mixed to a PDA agar medium (Difco) and thereafter cultured at 30°C for 2 days, and the viable bacterial count was measured.

[Table 5]

| <Microorganism:*Rhodotorula mucilaginosa*> | | | | | | | | | | |
|---|---|---|---|---|---|---|---|---|---|---|
| | | | Example | | | | | | Comparative example | Control |
| | | | 5-1 | 5-2 | 5-3 | 5-4 | 5-5 | 5-6 | 5-1 | |
| Metabolism controlling agent | | | C9OH | C10OH | C12OH | C14OH | C16OH | C12E1 | C18OH | |
| Metabolism suppression rate (%) | Amount of metabolism controlling agent **(μg/fabric g)** | 250 | 14 | 51 | 68 | 14 | 24 | 24 | 2 | 0 |
| Metabolite suppression rate (%) | Amount of metabolism controlling agent **(μg/fabric g)** | 250 | 21 | 46 | 62 | 45 | 38 | 41 | - | 0 |
| Bacterial count (Log (cfu/ fabric $cm^2$)) | Amount of metabolism controlling agent **(μg/fabric g)** | 250 | - | - | 4.9 | - | - | - | - | 4.9 |

<Example 6 and comparative example 6>

In the same manner as in each of examples 1 to 5 except that the amount of a metabolism controlling agent was as shown in Table 6, the metabolism of 14-methylhexadecanoic acid in each of the microorganisms was tested. The results are shown in Table 6. Note that, in this example, C12OH (1-dodecanol) was used as the metabolism controlling agent.

[Table 6]

| | | | Comparative example | Example | | | | | |
|---|---|---|---|---|---|---|---|---|---|
| | | | 6-1 | 6-1 | 6-2 | 6-3 | 6-4 | 6-5 | 6-6 |
| **Amount of metabolism controlling agent (μg/fabric g)** | | | 0 | 100 | 250 | 500 | 1000 | 2000 | 3000 |
| Microorganism | *Moraxella osloensis* | Metabolism suppression rate (%) | 0 | 23.8 | 79.4 | 83.3 | - | - | - |
| | | Metabolite suppression rate (%) | 0 | 33 | 76 | 85 | - | - | - |
| | | Bacterial count (Log (cfu/fabric $cm^2$)) | 6.0 | 6.0 | 6.5 | 5.6 | - | - | - |
| | *Micrococcus aloeverae* | Metabolism suppression rate (%) | 0 | 16.7 | 55.0 | 85.8 | 86.9 | - | - |
| | | Metabolite suppression rate (%) | 0 | 21 | 74 | 75 | 75 | - | - |
| | | Bacterial count (Log (cfu/fabric $cm^2$)) | 6.5 | 6.5 | 6.4 | 5.6 | 5.6 | - | - |
| | *Staphylococcus aureus* | Metabolite suppression rate (%) | 0 | 10.00 | 64.10 | 93.20 | - | - | - |
| | | Bacterial count (Log (cfu/fabric $cm^2$)) | 6.4 | 6.4 | 6.3 | 6.2 | - | - | - |
| | *Escherichia coli* | Metabolism suppression rate (%) | 0 | - | - | 21 | 61 | 66 | 73 |
| | | Metabolite suppression rate (%) | 0 | - | - | 41 | 65 | 77 | 80 |
| | | Bacterial count (Log (cfu/fabric $cm^2$)) | 6.4 | - | - | 6.3 | 6.4 | 6.3 | 6.3 |
| | *Rhodotorula mucilaginosa* | Metabolite suppression rate (%) | 0 | - | 72.50 | 65.80 | 82.20 | - | - |
| | | Bacterial count (Log (cfu/fabric $cm^2$)) | 4.9 | - | 4.9 | 4.7 | 4.8 | - | - |

<Example 7 and comparative example 7>

The suppression of a metabolite from 13-methyltetradecanoic acid by bacteria of the genus Staphylococcus (Staphylococcus epidermidis and Staphylococcus aureus) was tested in the following manner. The results are shown in Table 7.

(1) Suppression of metabolism of 13-methyltetradecanoic acid

Staphylococcus epidermidis was precultured on an SCD agar medium (Wako Pure Chemical Corporation) at 37°C for 18 hours, and a bacterial suspension of $10^{10}$ (CFU/ml) was prepared by using an SCD liquid medium (Wako Pure Chemical Corporation) diluted to 1/10 with ion exchange water.

A methanol solution in which 13-methyltetradecanoic acid (Larodan Fine Chemicals) was dissolved was added dropwise to 3 cm×3 cm pieces of sterilized plain-weave cotton fabric to make it adhered to the fabric. The adhesion amount of 13-methyltetradecanoic acid was set to 10 μg per piece of the plain-weave cotton fabric (100 μg/fabric g). Further, a methanol solution in which a metabolism controlling agent was dissolved was prepared and added dropwise

to the fabric to which 13-methyltetradecanoic acid adhered, and it was thereafter dried for an hour. The methanol solution was added dropwise such that the amount of the metabolism controlling agent was as shown in the table. This fabric was placed in a sterilized screw tube bottle No. 3 (Maruemu Corporation) and 100 μl of the bacterial suspension was inoculated thereto, and culture was carried out under 37°C conditions for 48 hours.

(2) Suppression of metabolite

3 ml of methanol was added to the screw tube bottle after culture, and extraction was carried out under ultrasonic irradiation for 30 minutes (20°C). The extraction liquid was mixed with (a 1000 ppm methanol solution of) an ADAM reagent of Funakoshi Co., Ltd. at 1:1 (volume ratio) and left overnight under room temperature and dark place conditions. After that, isovaleric acid as a metabolite from 13-methyltetradecanoic acid was quantified by HPLC. The quantification conditions are as follows.

Quantification conditions for metabolite

LC: Alliance HPLC e2695
Column: Zorbax C8 4.6x250 mm
Eluent: acetonitrile 61% (v/v), water 39% (v/v)
Column temperature: 40°C
Sample injection volume: 30 μL
Flow rate: 1.0 mL/min
Detection: FLD Ex. 365 nm, Em. 412 nm

The production amount of isovaleric acid after culture on the fabric to which the metabolism controlling agent was added was quantified, and the ratio of the production amount to that of isovaleric acid in the case of adding no metabolism controlling agent (control) was calculated as a metabolite suppression rate (%). Note that the metabolite suppression rate was determined by the following formula:

$$\text{Metabolite suppression rate (\%)} = 100 \times [(D-C)/D]$$

C: production amount of metabolite of example or comparative example (μg/fabric g)
D: production amount of metabolite of control (μg/fabric g)

(3) Measurement of viable bacterial count

9 mL of an LP diluent (manufactured by NIHON PHARMACEUTICAL CO., LTD.) was added to the fabric on which culture was carried out in the above (1), and the extraction of the bacteria was carried out under ultrasound for 10 minutes. The extraction liquid was cultured on an SCD-LP agar medium (NIHON PHARMACEUTICAL CO., LTD.) at 37°C for a day. The number of colonies obtained on each piece of the fabric was counted, and a common logarithm value thereof was measured as the viable bacterial count. A difference between logarithm value (1) of the viable bacterial count of the microorganism when no metabolism controlling agent is added and logarithm value (2) of the viable bacterial count of the microorganism when a metabolism controlling agent is added, logarithm value (1)-logarithm value (2), being -1 or more and less than 2 means that the viable bacterial count does not change regardless of the presence or absence of the contact of a metabolism controlling agent.

[Table 7]

| | | | Comparative example | Example | | |
|---|---|---|---|---|---|---|
| | | | 7-1 | 7-1 | 7-2 | 7-3 |
| **Amount of metabolism controlling agent (μg/fabric g)** | | | 0 | 50 | 100 | 500 |
| Microorganism | *Staphylococcus epidermidis* | Metabolite suppression rate (%) | 0 | 49.1 | 40.7 | 50.0 |
| | | Bacterial count (Log (cfu/fabric $cm^2$)) | 5.8 | 5.6 | 5.4 | 5.0 |

The compounds used in examination example are as follows.

[Metabolism controlling agent]

<Component (a) >

C12OH: 1-dodecanol

<Component (b) >

- Diclosan
- Triclosan
- Cerulenin

<Component (b'): comparative compound for component (b)>

• CCCP: carbonylcyanide-m-chlorophenylhydrazone

CCCP is known as an uncoupling agent and is a compound which does not inhibit fatty acid synthases.

<Branched fatty acid>

14-methylhexadecanoic acid: manufactured by Sigma-Aldrich Co. LLC

<Examination example>

The suppression of metabolism of 14-methylhexadecanoic acid by bacteria of the genus Moraxella and the suppression of a metabolite therefrom were tested in the following manner. The results are shown in Tables 8 to 11.

(1) Suppression of metabolism of 14-methylhexadecanoic acid

Moraxella osloensis (strain isolated from clothing) was precultured on an SCD-LP agar medium (NIHON PHARMACEUTICAL CO., LTD.) at 37°C for 24 hours, and a bacterial suspension of $10^6$ (CFU/ml) was prepared by using an NB liquid medium (Difco) diluted to 1/20 with ion exchange water.

A methanol solution in which 14-methylhexadecanoic acid (SIGMA-ALDRICH) was dissolved was added dropwise to 3 cmx3 cm pieces of sterilized plain-weave cotton fabric to make it adhered to the fabric. The adhesion amount of 14-methylhexadecanoic acid was set to 10 μg per piece of the plain-weave cotton fabric (100 μg/fabric g). Further,

a methanol solution in which component (a) was dissolved, and then, a methanol solution in which component (b) or (b') was dissolved were added dropwise to the fabric to which 14-methylhexadecanoic acid adhered, and it was thereafter dried for an hour. The methanol solutions were added dropwise such that the amount of component (a) or (b) was as shown in the tables. This fabric was placed in a sterilized screw tube bottle No. 3 (Maruemu Corporation) and 100 μl of the bacterial suspension was inoculated thereto, and culture was carried out under 37°C conditions for 21 hours.

3 ml of methanol was added to the screw tube bottle after culture, and extraction was carried out under ultrasonic irradiation for 30 minutes (20°C). The extraction liquid was mixed with a (trimethylsilyl)diazomethane hexane solution (FUJIFILM Wako Pure Chemical Corporation) at 9:1 (volume ratio) and left overnight under room temperature and dark place conditions. After that, 14-methylhexadecanoic acid remaining on the fabric was quantified by GC. The quantification conditions for GC are as follows.

- GC: Agilent 6890N
- GC column: Agilent DB-1 30 mx250 μm×0.25 μm
- Temperature rise condition: 50°C (held for 3 minutes)-(10°C/min)-300°C (held for 5 minutes)
- Sample injection volume: 1 μl
- Split ratio: 1:12
- Detector: FID

The residual amount of 14-methylhexadecanoic acid after culture on the fabric to which each metabolism controlling agent was added was quantified, and the ratio of the residual amount to that of 14-methylhexadecanoic acid in the case of adding no metabolism controlling agent (control) was calculated as a metabolism suppression rate (%). The higher a numerical value thereof is, the more the metabolism is suppressed. Note that the metabolism suppression rate was determined by the following formula:

$$\text{Metabolism suppression rate } (\%) = [1-(100-A)/(100-B)]\times 100$$

A: residual amount of 14-methylhexadecanoic acid of reference or examination example (μg/fabric g)
B: residual amount of 14-methylhexadecanoic acid of control (μg/fabric g)

A control is an example in which none of component (a), (b) or (b') is added, and references 1-1, 2-1, 3-1 and 4-1 qualify as this (the same applies hereinafter). In these controls, the metabolism suppression rate is 0%.

(2) Suppression of metabolite

After culture on fabric prepared in the same manner as in the above (1), 3 ml of methanol was added, and extraction was carried out under ultrasonic irradiation for 30 minutes (20°C). The extraction liquid was mixed with (a 1000 ppm methanol solution of) an ADAM reagent of Funakoshi Co., Ltd. at 1:1 (volume ratio) and left overnight under room temperature and dark place conditions. After that, 4-methyl-3-hexenoic acid as a metabolite from 14-methylhexadecanoic acid was quantified by HPLC. The quantification conditions are as follows.

Quantification conditions for metabolite

LC: HITACHI ELITE LaChrom
Column: Zorbax C8 4.6×250 mm
Eluent: acetonitrile 61% (v/v), water 39% (v/v)
Column temperature: 40°C
Sample injection volume: 10 μL
Flow rate: 1.0 mL/min
Detection: FLD Ex. 365 nm, Em. 412 nm

The production amount of 4-methyl-3-hexenoic acid after culture on the fabric to which the metabolism controlling agent was added was quantified, and the ratio of the production amount to that of 4-methyl-3-hexenoic acid in the case of adding no metabolism controlling agent (control) was calculated as a metabolite suppression rate (%). The higher a

numerical value thereof is, the more the metabolism is suppressed. Note that the metabolite suppression rate was determined by the following formula. In references 1-1, 2-1, 3-1 and 4-1, which are controls, the metabolite suppression rate is 0%.

$$\text{Metabolite suppression rate (\%)} = 100 \times [(D-C)/D]$$

C: production amount of metabolite of reference or examination example (μg/fabric g)
D: production amount of metabolite of control (μg/fabric g)

(3) Measurement of viable bacterial count

After culture on fabric prepared in the same manner as in the above (1), 9 mL of an LP diluent (manufactured by NIHON PHARMACEUTICAL CO., LTD.) was added, and the extraction of the bacteria was carried out under ultrasound for 10 minutes. The extraction liquid was poured and mixed to an SCD-LP agar medium (NIHON PHARMACEUTICAL CO., LTD.), and culture was thereafter carried out at 37°C for a day. The number of colonies obtained on each piece of the fabric was counted, and a common logarithm value thereof was measured as the viable bacterial count. A difference between logarithm value (1) of the viable bacterial count of the microorganism when no metabolism controlling agent is added and logarithm value (2) of the viable bacterial count of the microorganism when a metabolism controlling agent is added, logarithm value (1)-logarithm value (2), being -1 or more and less than 2 means that the viable bacterial count does not change regardless of the presence or absence of the contact of a metabolism controlling agent and the viable bacterial count of the microorganism is maintained. In addition, the closer a value thereof is to 0, the more the viable bacterial count of the microorganism is maintained.

The results of examination examples 1 to 3 are shown in Tables 8 to 10. In addition, Table 11 shows the result of examination example 4 in which carbonylcyanide-m-chlorophenylhydrazone of component (b'), which is an uncoupling agent, was used instead of component (b).

[Table 8]

| | Metabolism controlling agent | | Metabolism of branched fatty acid | | Product of metabolism of branched fatty acid | | Viable bacterial count (Log (cfu/fabric $cm^2$)) |
|---|---|---|---|---|---|---|---|
| | (a) | (b) | | | | | |
| | C12OH **(μg/ fabric g)** | Diclosan **(μg/ fabric g)** | Residual amount **(μg/fabric g)** | Metabolism suppression rate (%) | Production amount **(μg/ fabric g)** | Metabolite suppression rate (%) | |
| Reference 1-1 | 0 | 0 | 39 | 0 | 4.4 | 0 | 6.4 |
| Reference 1-2 | 0 | 1 | 43 | 7 | 4.2 | 5 | 6.2 |
| Reference 1-3 | 0 | 2 | 46 | 11 | 3.8 | 14 | 6.0 |
| Reference 1-4 | 0 | 5 | 56 | 27 | 2.6 | 41 | 5.1 |
| Reference 1-5 | 100 | 0 | 59 | 33 | 3.5 | 20 | 6.2 |
| Examination example 1-1 | 100 | 1 | 76 | 61 | 2.5 | 43 | 6.0 |
| Examination example 1-2 | 100 | 2 | 91 | 85 | 2.1 | 52 | 6.3 |
| Examination example 1-3 | 100 | 5 | 93 | 89 | 1.6 | 64 | 5.2 |

[Table 9]

| | Metabolism controlling agent | | Metabolism of branched fatty acid | | Product of metabolism of branched fatty acid | | Viable bacterial count (Log (cfu/fabric $cm^2$)) |
|---|---|---|---|---|---|---|---|
| | (a) | (b) | Residual amount **(μg/fabric g)** | Metabolism suppression rate (%) | Production amount **(μg/ fabric g)** | Metabolite suppression rate (%) | |
| | C12OH **(μg/ fabric g)** | Triclosan **(μg/ fabric g)** | | | | | |
| Reference 2-1 | 0 | 0 | 39 | 0 | 4.4 | 0 | 6.4 |
| Reference 2-2 | 0 | 0.5 | 43 | 6 | - | - | 6.2 |
| Reference 2-3 | 0 | 1 | 38 | -2 | 4.2 | 5 | 6.2 |
| Reference 2-4 | 0 | 2 | 44 | 8 | 4.1 | 7 | 6.0 |
| Reference 2-5 | 100 | 0 | 59 | 33 | 3.5 | 20 | 6.2 |
| Examination example 2-1 | 100 | 0.5 | 71 | 53 | - | - | 6.2 |
| Examination example 2-2 | 100 | 1 | 80 | 67 | 2.3 | 48 | 6.2 |
| Examination example 2-3 | 100 | 2 | 98 | 97 | 1.9 | 57 | 6.5 |

[Table 10]

| | Metabolism controlling agent | | Metabolism of branched fatty acid | | Product of metabolism of branched fatty acid | | Viable bacterial count (Log (cfu/fabric $cm^2$)) |
|---|---|---|---|---|---|---|---|
| | (a) | (b) | Residual amount **(μg/fabric g)** | Metabolism suppression rate (%) | Production amount **(μg/fabric g)** | Metabolite suppression rate (%) | |
| | C12OH **(μg/ fabric g)** | Cerulenin **(μg/ fabric g)** | | | | | |
| Reference 3-1 | 0 | 0 | 39 | 0 | 4.4 | 0 | 6.4 |
| Reference 3-2 | 0 | 5 | 39 | 0 | 4.3 | 2 | 6.1 |
| Reference 3-3 | 0 | 10 | 41 | 3 | 4.0 | 9 | 6.3 |
| Reference 3-4 | 0 | 20 | 48 | 15 | 3.9 | 11 | 6.3 |
| Reference 3-5 | 100 | 0 | 59 | 33 | 3.5 | 20 | 6.2 |
| Examination example 3-1 | 100 | 5 | 74 | 57 | 2.4 | 45 | 6.1 |

(continued)

| | Metabolism controlling agent | | Metabolism of branched fatty acid | | Product of metabolism of branched fatty acid | | Viable bacterial count (Log (cfu/fabric cm$^2$)) |
|---|---|---|---|---|---|---|---|
| | (a) | (b) | Residual amount **(μg/fabric g)** | Metabolism suppression rate (%) | Production amount **(μg/fabric g)** | Metabolite suppression rate (%) | |
| | C12OH **(μg/ fabric g)** | Cerulenin **(μg/ fabric g)** | | | | | |
| Examination example 3-2 | 100 | 10 | 89 | 82 | 2.2 | 50 | 5.9 |
| Examination example 3-3 | 100 | 20 | 84 | 74 | 1.5 | 66 | 6.1 |

[Table 11]

| | Metabolism controlling agent | | Metabolism of branched fatty acid | | Product of metabolism of branched fatty acid | | Viable bacterial count (Log (cfu/fabric cm$^2$)) |
|---|---|---|---|---|---|---|---|
| | (a) | (b') | Residual amount (μg/fabric g) | Metabolism suppression rate (%) | Production amount (μg/ fabric g) | Metabolite suppression rate (%) | |
| | C12OH **(μg/ fabric g)** | CCCP **(μg/ fabric g)** | | | | | |
| Reference 4-1 | 0 | 0 | 39 | 0 | 4.4 | 0 | 6.4 |
| Reference 4-2 | 0 | 2 | 44 | 8 | 3.3 | 25 | 6.1 |
| Reference 4-3 | 0 | 5 | 56 | 28 | 2.8 | 36 | 5.5 |
| Examination example 4-1 | 100 | 0 | 59 | 33 | 3.5 | 20 | 6.2 |
| Examination example 4-2 | 100 | 2 | 61 | 36 | 2.4 | 45 | 5.8 |
| Examination example 4-3 | 100 | 5 | 73 | 55 | 1.5 | 66 | 5.4 |

Tables 8 to 11 show results when using component (a) or (b) alone and when using components (a) and (b) together. In the tables, having a metabolism suppression rate or metabolite suppression rate synergistically increased due to the combined use of components (a) and (b) is preferable as it means being superior in the effect of controlling the metabolism of a branched fatty acid.

In the above tables, for example, in Table 8, reference 1-1, in which the branched fatty acid and the microorganism coexist in their respective predetermined amounts on the textile product used as an exemplary object, uses neither C12OH nor diclosan as a metabolism controlling agent, and has a metabolism suppression rate and a metabolite suppression rate of both 0%.

In Table 8, the values of reference 1-2 are those when diclosan coexists, but C12OH does not coexist. The values of reference 1-5 are those when diclosan does not coexist, but C12OH coexists. The values of examination example 1-1 are those when both diclosan and C12OH coexist. Here, reference to the metabolism suppression rate of the branched fatty acid shows that, while the total of the values of reference 1-2 (component (b) alone) and reference 1-5 (component (a) alone) is 7%+33%, which is 40%, the value of the examination example 1-1 (components (a) and (b)) qualifying as an example is 61% and is higher than 40% of the above total, resulting in a higher value due to the synergistic effect of diclosan and C12OH. The same synergistic improvement can also be seen in the metabolite suppression rate of examination example 1-1. The same applies to examination examples 1-2 and 1-3 qualifying as examples, in which the synergistic improvement can be seen in the metabolism suppression rates and metabolite suppression rates.

The same synergistic improvement can also be seen in the metabolism suppression rates and metabolite suppression rates of the examination examples in Tables 9 to 10 qualifying as examples.

## Claims

1. A method for controlling the metabolism of a branched fatty acid comprising, bringing (a) one or more compounds selected from a compound represented by the following general formula (1) into contact with a microorganism to control the metabolism of a branched fatty acid of the microorganism,

   $$RO\text{-}(C_2H_4O)_n\text{-}H \qquad (1)$$

   wherein R is a linear aliphatic hydrocarbon group with 9 or more and 16 or less carbons, and n is 0 or 1.

2. A method for controlling the metabolism of a branched fatty acid comprising, bringing (a) one or more compounds selected from a compound represented by the following general formula (1) [hereinafter referred to as component (a)] and (b) an inhibiting agent for fatty acid synthases [hereinafter referred to as component (b)] into contact with a microorganism to control the metabolism of a branched fatty acid of the microorganism,

   $$RO\text{-}(C_2H_4O)_n\text{-}H \qquad (1)$$

   wherein R is a linear aliphatic hydrocarbon group with 9 or more and 16 or less carbons, and n is 0 or 1.

3. The method for controlling the metabolism of a branched fatty acid according to claim 1, wherein a viable bacterial count of the microorganism is substantially maintained before and after the contact of the component (a).

4. The method for controlling the metabolism of a branched fatty acid according to claim 1 or 2, wherein the component (a) is brought into contact with the microorganism under the presence of a branched fatty acid.

5. The method for controlling the metabolism of a branched fatty acid according to claim 2, wherein a viable bacterial count of the microorganism is substantially maintained before and after the contact of the components (a) and (b).

6. The method for controlling the metabolism of a branched fatty acid according to claim 2 or 5, wherein the components (a) and (b) are brought into contact with the microorganism under the presence of a branched fatty acid.

7. The method for controlling the metabolism of a branched fatty acid according to claim 2, 5 or 6, wherein a mass ratio between a use amount of the component (a) and a use amount of the component (b), (b)/(a), is 0.001 or more and 1 or less.

8. The method for controlling the metabolism of a branched fatty acid according to any of claims 1 to 7, wherein the metabolism of the branched fatty acid of the microorganism is β oxidization.

9. An agent for controlling the metabolism of a branched fatty acid in a microorganism comprising, (a) one or more compounds selected from a compound represented by the following general formula (1),

   $$RO\text{-}(C_2H_4O)_n\text{-}H \qquad (1)$$

   wherein R is a linear aliphatic hydrocarbon group with 9 or more and 16 or less carbons, and n is 0 or 1.

10. An agent for controlling the metabolism of a branched fatty acid in a microorganism comprising, (a) one or more compounds selected from a compound represented by the following general formula (1) and (b) an inhibiting agent for fatty acid synthases,

    $$RO\text{-}(C_2H_4O)_n\text{-}H \qquad (1)$$

    wherein R is a linear aliphatic hydrocarbon group with 9 or more and 16 or less carbons, and n is 0 or 1.

**11.** The agent for controlling the metabolism of a branched fatty acid according to claim 9 or 10, wherein the metabolism of the branched fatty acid of the microorganism is β oxidization.

# INTERNATIONAL SEARCH REPORT

International application No.
PCT/JP2020/043557

A. CLASSIFICATION OF SUBJECT MATTER
C12N 1/20(2006.01)i; C12N 1/16(2006.01)i
FI: C12N1/20 Z; C12N1/16 Z
According to International Patent Classification (IPC) or to both national classification and IPC

B. FIELDS SEARCHED
Minimum documentation searched (classification system followed by classification symbols)
C12N1/20; C12N1/16

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2009-78987 A (KAO CORP.) 16 April 2009 (2009-04-16) claims, example 1 | 9, 11<br>1-8, 10 |
| X<br>A | JP 2018-52980 A (KAO CORP.) 05 April 2018 (2018-04-05) claims, examples | 9, 11<br>1-8, 10 |
| A | JP 2012-127012 A (KAO CORP.) 05 July 2012 (2012-07-05) claims, examples | 1-11 |
| A | JP 7-509470 A (THE JOHNS HOPKINS UNIVERSITY) 19 October 1995 (1995-10-19) claims | 1-11 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 January 2021 (14.01.2021) | 26 January 2021 (26.01.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/043557

| Patent Documents referred in the Report | Publication Date | Patent Family | Publication Date |
|---|---|---|---|
| JP 2009-78987 A | 16 Apr. 2009 | (Family: none) | |
| JP 2018-52980 A | 05 Apr. 2018 | JP 2015-28128 A | |
| JP 2012-127012 A | 05 Jul. 2012 | (Family: none) | |
| JP 7-509470 A | 19 Oct. 1995 | US 5665874 A claims<br>EP 651636 A1<br>WO 1994/002108 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012127012 A
- WO 2009037861 A
- JP 2009078987 A
- WO 9402108 A
- US 5539132 B
- US 5981575 B

### Non-patent literature cited in the description


- **DILS et al.** *Meth. Enzymol.,* 1975, vol. 35, 74-83
- **WANG et al.** *Tetrahedron Lett.,* 1984, vol. 25, 5243-5246
- **OISHI et al.** *J. Antibiotics,* 1982, vol. 35, 391-395
- **KREMER et al.** *J. Bio. Chem.,* 2000, vol. 275, 16857-16864
- **MORISAKI et al.** *Chem. Pharm. Bull.,* 1992, vol. 40, 2945-2953
- **SHIMAZAWA et al.** *Chem. Pharm. Bull.,* 1992, vol. 40, 2954-2957